# EUROPEAN PATENT APPLICATION

(11) **EP 2 400 303 A1**
(43) Date of publication of application: **28.12.2011**
(21) Application number: 10167341.6
(22) Date of filing: 25.06.2010
(51) Int. Cl.: G01N 33/66, G01N 33/68

(54) **Markers of Oocyte quality**

(71) Applicant: UNIVERSITY COLLEGE DUBLIN, Dublin 4 (IE)
(72) Inventor: Brennan, Lorraine, 4 Dublin (IE); Wallace, Martina, 4 Dublin (IE); Wingfield, Mary, 2 Dublin (IE); Cottell, Evelyn, 2 Dublin (IE)
(74) Representative: Gates, Marie Christina Esther

(57) **Abstract**

A method of increasing the success rate of IVF comprising selection of an oocyte for fertilisation, or of an embryo for implantation, based on the metabolite concentration of the follicular fluid surrounding the oocyte.

## Description

### Field of the Invention

The present invention relates to markers of oocyte quality, to methods of assessing the fertilisation potential of oocytes and their development potential as well as diagnostic kits suitable for use in such methods. The markers, methods and kits have an important role in fertility treatment.

### Background to the Invention

A major reason that women seek assisted reproductive technologies is for treatment of reduced fertility. Generally a woman's age is the single most significant factor in predicting fertility success, but even among women of the same age, fertilization ability varies, as does the fertilization ability of the oocytes isolated from the same woman. For this reason women undergoing ART often endure multiple failed attempts at pregnancy, with is both costly and emotionally draining.

The process of in vitro fertilisation (IVF) involves removal of ova from follicles, together with some of the follicular fluid surrounding them. The ova are then exposed to sperm in vitro and the resulting embryos are returned to the uterus for implantation. Is it not possible to determine by visual examination if an ova is likely to undergo fertilization and so it is common procedure to remove several ova and to attempt to fertilize all of them. There is no simple or convenient method by which one can determine which of the removed ova is most likely to be capable of development. It would thus be highly advantageous to be able to determine which ova have a high probabability of fertilization, to fertilize those ova and then to implant only them. Apart from overcoming ethical considerations, it would increase the chances of successful pregnancy. Follicular fluid composition and follicular somatic cells play an important role in the maturation of the developing oocyte within the female ovary. There is thus a need to identify follicle-specific markers of ovarian function that could be successfully used in the diagnosis and treatment of fertility disorders.

In today's society, assistive reproductive techniques (ART) have become widespread and the latest figures show that they account for the birth of more than 3 million babies worldwide. One of the most serious complications of in vitro fertilization (IVF) treatment is a high multiple pregnancy rate of approximately 30%, which leads to a greater incidence of medical complications. Multiple pregnancies have an increased incidence of prenatal morbidity and mortality, mainly due to the risk of prematurity, which is 20% in twin pregnancies and 50% in triplet pregnancies. In an effort to reduce this high multiple pregnancy rate, elective single embryo transfer is advocated, rather than the more standard transfer of 2 embryos. However, pregnancy success rates are relatively low with single embryo transfer and there is a lack of an accurate method to assess embryo viability potential, thus limiting its acceptance. At present morphological assessment remains the primary technique for evaluation of viable embryos for transfer, during IVF cycles. Morphological assessment has modest predictive power and so the development of new selection methods is required.

Currently used processes of metabolomic profiling of embryo culture media have been performed using NIR and Raman Spectroscopy. One of the main problems with this approach is it requires fertilization of all oocytes and the reselection is performed at the embryo stage.

Follicular fluid is routinely discarded at oocyte collection during IVF treatment. The present studies have revealed that the metabolic profile of follicular fluid can predict oocyte quality and pregnancy outcome. The metabolic profile is obtained using 1H NMR and contains information about metabolite levels in the follicular fluid. Using this profile in combination with multivariate data analysis, it is possible to predict oocyte quality and pregnancy outcome.

The advantage of the current invention is that it uses follicular fluid for the analysis, which can be collected in abundance and is normally a waste fluid in the IVF clinic. The metabolomic profile can then be used in combination with morphology to select oocytes for fertilization and to select embryos for implantation. This in turn allows single embryo transfer and reduces the number of embryos formed and stored.

### Object of the Invention

It is thus an object of the invention to provide markers and methods that can be used to predict which oocytes should be fertilized, based on fertilisation potential, thus reducing the necessity of fertilizing all oocytes. This in turn would reduce the storage and ethical problems associated with frozen embryos. A further object of the invention is to provide markers and methods for determining the viability potential of the embryo, based on analysis of the follicular fluid surrounding the ovum. By selecting the ova with the best potential for developing into a full term pregnancy, the IVF success rate could be increased.

### Summary of the Invention

According to the present invention there is provided a method of increasing the success rate of IVF comprising selection of an oocyte for fertilisation or of an embryo for implantation based on the metabolite concentration of the follicular fluid surrounding the oocyte wherein the metabolite is selected from the group comprising lactate, glutamine, choline/phospho-choline, alanine, pyruvate, valine, glucose, proline, leucine or isoleucine.

In one embodiment the invention provides a method of determining the fertilization potential of an ooctye comprising :
(1) taking a sample of follicular fluid from an oocyte in vitro,
(2) determining the lactate and glutamine concentration of the follicular fluid,
(3) calculating the ratio of lactate concentration to glutamine concentration,
(4) comparing the ratio of lactate concentration to glutamine concentration to a standard to determine if an oocyte has an increased potential for fertilisation.

The standard may be either an average ratio based on the ratio of concentrations from a plurality of oocytes which are not capable of fertilisation, or an average ratio based on the ratio of concentrations from a plurality of oocytes which are capable of fertilisation. The ratio of lactate concentration to glutamine concentration of an oocyte which will fertilise may be in the range 11.13 +/- 3.21.

By increased potential for fertilisation is meant that the oocyte will fertilise.

The invention also relates to a method of determining whether an embryo will develop past the one cell stage comprising :
(1) taking a sample of follicular fluid from an oocyte in vitro,
(2) determining the concentration of the follicular fluid of one or more of lactate, choline/phospho-choline, alanine, pyruvate, valine, isoleucine or glucose
(3) comparing the concentrations of lactate, choline/phospho-choline, alanine, pyruvate, valine, isoleucine or glucose to a standard, wherein a decrease in the concentration of glucose or an increase in the concentration of lactate or choline/phospho-choline or alanine or pyruvate or valine or isoleucine when compared to the standard, is indicative of an oocyte that will develop past the one cell stage.

The standard may be either an average concentration based on the concentrations from a plurality of embryos which will develop past the one cell stage, or an average concentration based on the concentrations from a plurality of embryos which will not develop past the one cell stage.

The concentration of lactate, choline/phospho-choline, alanine, pyruvate, valine, isoleucine or glucose of an embryo which will develop past the one cell stage may be as shown in Table 3.

The invention also relates to a method of determining whether an embryo will implant:
(1) taking a sample of follicular fluid from an oocyte in vitro,
(2) determining the concentration of the follicular fluid of one or more of glucose, proline, lactate, leucine, isoleucine or glutamine,
(3) comparing the glucose, proline, lactate, leucine, isoleucine or glutamine concentrations to a standard, wherein a decrease in the concentration of glucose or glutamine or an increase in the concentration of proline or lactate or leucine or isoleucine when compared to the standard, is indicative of a positive pregnancy outcome.

The standard may be either an average concentration based on the concentrations from a plurality of embryos which will implant, or an average concentration based on the concentrations from a plurality of embryos which will not will implant.

The concentration of glucose, proline, lactate, leucine or isoleucine of an embryo will implant may be as shown in Table 2. The concentration of glutamine may be as shown in Table 5.

In a further aspect the invention provides a diagnostic kit for the determination of the fertilization potential of an oocyte comprising reagents for the determination of the lactate to glutamine concentration. Such reagents are known in the art.

The invention also provides use of the concentration of one or more of the metabolites lactate, choline/phosphochoine, alanine, pyruvate, valine, isoleucine and glucose in a method of determining the ability of the embryo to develop past the one cell stage. The use may involve the determination of the relative concentrations of groups of the metabolites.

The invention also provides use of the concentration of one or more of the metabolites lactate, proline, isoleucine/leucine and glucose in a method of determining the ability of the embryo to implant. The use may involve the determination of the relative concentrations of groups of the metabolites.

There are many techniques known in the art for the measurement of concentrations of metabolites in bodily fluids. NMR analysis could be used. A Ysi analyser may be used to measure glutamine, glucose, lactate and glutamate. Wallace et al 2010 Human reproduction 25(4) : 949-56 describes such methods.

### Brief Description of the Drawings

Figure 1. PLS-DA plot of follicular fluid samples from follicles where the oocyte resulted in a 1 cell embryo (n=9, black box) or a 3 cell embryo (n=13, blue diamond) on day 2 after fertilisation. r2x=0.515, r2y=0.702, q2= 0.478.
Figure 2. PLS-DA plot of follicular fluid from follicles where the tracked oocyte resulted in an embryo that was transferred where the patient became pregnant (class 3, n=10), or was did not become pregnant (class 4, n=12). 2 component model, r2=0.517, q2= 0.12.
Figure 3. PLS-DA scores plot obtained from the metabolomic profile of follicular fluid. Red diamonds represent pregnant subjects (n=10) and class 4 represent non-pregnant (n=12) subjects.

### Detailed Description of the Drawings

### Results

A total of 110 follicular fluid samples were collected from 59 patients undergoing IVF treatment. Ten of these patients gave one follicular fluid sample, 47 patients gave 2 follicular fluid samples and 2 patients gave 3 follicular fluid samples. The average age of the patients was 37 ± 3 years. Out of the 110 follicular fluid samples collected, 38 of the tracked oocytes from these follicles failed to fertilise, while 40 tracked oocytes went on to form embryos that were transferred back to the womb during the IVF cycle. Thirteen of the patients, where a tracked oocyte was transferred, became pregnant.

### Developmental potential of the tracked oocyte

The metabolite composition of follicular fluid where the oocyte failed to fertilise successfully was investigated and a significant difference in the ratio of lactate to glutamine concentration in the follicular fluid from this group (9.19 ± 2.51) and the follicular fluid from the group termed transferred pregnant (11.13 ± 3.21) was found (p<0.007).

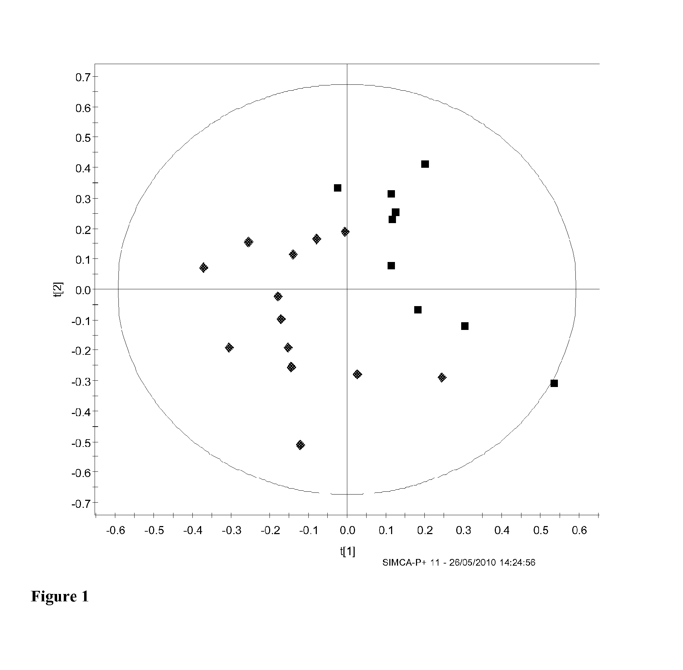

Follicular fluid samples were split into groups based on the cell number of the embryos that resulted from the tracked oocytes in order to further investigate links between follicular fluid composition and embryo development. Nine tracked oocytes formed a 1 cell embryo, 19 formed a 2 cell embryo, 13 formed a 3 cell embryo, 27 formed a 4 cell embryo and 5 formed a 4 cell embryo. PCA and PLS-DA of the 1H NMR spectra of the follicular fluid revealed differences in the metabolomic profile of follicular fluid from follicles where the oocyte resulted in a 1 cell embryo compared to those that formed a 2 cell, 3 cell or 4 cell embryo by day 2 following fertilisation. Robust models were built for pair wise comparisons with the comparison of 1 cell and 3 cell resulting in the strongest model. The PLS-DA model of these two groups is shown in figure 1 Analysis of the corresponding loadings and VIP plots revealed a decrease in levels of lactate, choline/phosphocholine, alanine, pyruvate, valine and isoleucine and an increase in levels of glucose and HDL/LDL in follicular fluid where the oocyte resulted in a 1 cell embryo (Table 3).

Analysis of follicular fluid samples grouped based on the morphological grade of the embryo that resulted was performed. Seventeen were grouped as grade 1 or 2a, 41 were grouped as grade 2b and 14 were grouped as grade 3, 4 or 5.Metabolic parameters that were found to be different between those graded 1 or 2a and 2b on the second day after fertilisation are shown in table 1.

**Table 1. Differences in the metabolite composition of follicular fluid where the oocyte resulted in a grade 1 and 2a or a grade 2b embryo. P value derived from general linear models using patient age as a covariate.**

| | Grade 2a and 1 (n=17) | Grade 2b (n=41) | P value |
|---|---|---|---|
| Glutamine | 0.40 ± 0.09 | 0.46 ± 0.09 | 0.043 |
| Lactate/glutamine | 11.14 ± 3.83 | 8.97 ± 2.48 | 0.008 |
| % Lactate | 62.99 ± 13.08 | 56.06 ± 11.19 | 0.040 |

### Pregnancy outcome of the tracked oocytes/embryos

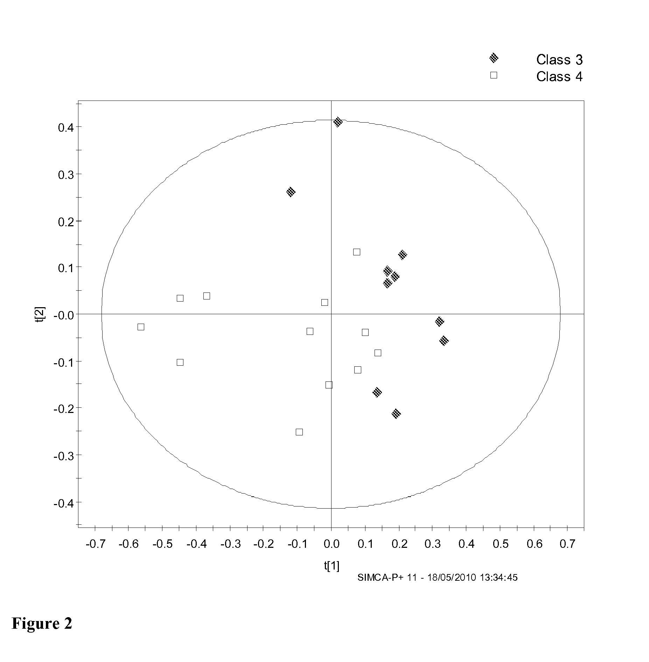

Analysis of follicular fluid based on whether the tracked oocyte resulted in a patient who became pregnant (n=13) or no pregnant (class 4, n=27) was performed. Prior to analysis, all occurrences where two tracked embryos were transferred back in the same patient were removed (n=2). Principal component analysis revealed 3 large outliers in the pregnant group which were subsequently removed and 14 samples from the transferred but unsuccessful group were removed in order to obtain near equal numbers in the groups for analysis. The PLS-DA model of these two groups is shown in figure 2. Analysis of the corresponding loadings and VIP plots revealed a decrease in levels of glucose and an increase in levels of proline lactate and leucine/isoleucine in follicular fluid where the tracked embryo was transferred in a successful pregnancy. Univariate statistical analysis of the bin regions confirmed that all metabolites were significantly different between the two groups (table 2).

**Table 2. Metabolites identified as being discriminant between follicular fluid where the tracked embryo was transferred and the patient became pregnant or didn't become pregnant. Average intensities of metabolite bin regions are shown and p values are derived from general linear models using age as a covariate.**

| Metabolite | ppm | Pregnant | Non-pregnant | P value |
|---|---|---|---|---|
| Glucose | 3.4 | 2.09 ± 0.54 | 2.69 ± 0.69 | 0.022 |
| Proline | 2 | 1.19 ± 0.11 | 1.06 ± 0.14 | 0.027 |
| Lactate | 1.36 | 9.02 ± 1.95 | 7.23 ± 1.89 | 0.041 |
| Leucine/Isoleucine | 0.92 | 1.22 ± 0.14 | 1.08 ± 0.09 | 0.009 |

**Table 3. Metabolites identified from ¹H NMR spectra as being discriminant between follicular fluid where the tracked oocyte resulted in a 1 cell embryo or a 3 cell embryo. Average intensities of the metabolite bin regions ± the standard deviation is shown.**

| Metabolite | ppm | 1 cell embryo | 3 cell embryo |
|---|---|---|---|
| Lactate | 1.32 | 9.31 ± 2.25 | 11.46 ± 3.40 |
| Glucose | 3.4 | 2.89 ± 0.98 | 2.42 ± 0.69 |
| Choline/phospho-choline | 3.2 | 2.47 ± 0.32 | 2.79 ± 0.43 |
| Alanine | 1.48 | 1.73 ± 0.37 | 2.00 ± 0.24 |
| Pyruvate | 2.36 | 1.18 ± 0.31 | 1.40 ± 0.21 |
| Valine | 1 | 1.00 ± 0.17 | 1.14 ± 0.19 |
| Leucine/Isoleucine | 0.96 | 1.32 ± 0.15 | 1.46 ± 0.19 |

Independent analysis of glucose, lactate and glutamine showed a significant difference in the concentration of lactate aswell as lactate and glucose levels when expressed as a % of the total concentration of metabolites analysed (Table 3).

**Table 4. Independent analysis of metabolites in the follicular fluid samples that were used in the PLS-DA model shown in figure 1. P values are derived from general linear models using age as a covariate.**

| | Pregnant (n=10) | Non-pregnant (n=12) | P value |
|---|---|---|---|
| Glucose | 2.23 ± 0.64 | 2.54 ± 0.59 | 0.121 |
| Lactate | 4.57 ± 0.95 | 3.58 ± 0.89 | 0.018 |
| Glutamine | 0.45 ± 0.09 | 0.43 ± 0.06 | 0.603 |
| Glucose/lactate | 0.54 ± 0.28 | 0.78 ± 0.36 | 0.058 |
| Glucose/glutamine | 5.09 ± 1.57 | 5.92 ± 1.16 | 0.075 |
| Lactate/glutamine | 10.62 ± 3.03 | 8.40 ± 2.19 | 0.072 |
| % Glucose | 31.06 ± 9.81 | 39.20 ± 9.55 | 0.039 |
| % Lactate | 62.73 ± 10.44 | 54.21 ± 9.97 | 0.043 |
| % Glutamine | 6.20 ± 1.35 | 6.59 ± 0.64 | 0.492 |

Figure 3 shows that the metabolomic profile of follicular fluid can correctly predict pregnancy outcome. The metabolomic profile of follicular can predict oocyte competence and thus may be used to screen oocytes for fertilization and to select embryos to transfer. Metabolites identified as significantly different between the two groups include those listed in Table 5 and Table 6.

**Table 5. Metabolite concentrations in follicular fluid measured using a YSI amino acid analyser.**

| | Pregnant (n=14) | Non-pregnant (n=26) | P value |
|---|---|---|---|
| % Glutamine | 5.66 ± 1.46 | 6.45 ± 1.08 | 0.008 |
| Glucose:glutamine | 6.68 ± 3.09 | 5.59 ± 2.13 | 0.027 |
| Lactate: glutamine | 11.17 ± 3.23 | 9.41 ± 2.65 | 0.011 |

The values are averages ± SD. The above metabolite and metabolite ratios are significantly different in follicular fluid from oocytes that resulted in a successful pregnancy and those that did not.

**Table 6. Metabolites obtained from NMR metabolomic analysis.**

| **Metabolite** | **NMR Region ppm** | **Pregnant** | **Non-pregnant** | **P value** |
|---|---|---|---|---|
| Glucose | 3.72 | 3.46 ± 0.65 | 4.51 ± 1.19 | 0.022 |
| Glucose | 3.48 | 3.32 ± 0.82 | 4.34 ± 1.18 | 0.031 |
| Glucose | 3.44 | 2.71 ± 0.59 | 3.41 ± 0.75 | 0.026 |
| Glucose | 3.4 | 2.09 ± 0.54 | 2.69 ± 0.69 | 0.035 |
| Glucose | 3.28 | 1.94 ± 0.10 | 2.07 ± 0.15 | 0.031 |
| Proline | 2.00 | 1.19 ± 0.11 | 1.06 ± 0.14 | 0.027 |
| Lactate | 1.36 | 9.02 ± 1.95 | 7.23 ± 1.89 | 0.041 |
| Leucine/Isoleucine | 0.92 | 1.22 ± 0.14 | 1.08 ± 0.09 | 0.009 |

Glucose gives numerous peaks in the NMR spectra. Values are averages ± SD and are relative intensities.

The words "comprises/comprising" and the words "having/including" when used herein with reference to the present invention are used to specify the presence of stated features, integers, steps or components but does not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. A method of increasing the success rate of IVF comprising selection of an oocyte for fertilisation or of an embryo for implantation based on the metabolite concentration of the follicular fluid surrounding the oocyte wherein the metabolite is selected from the group comprising lactate, glutamine, choline/phospho-choline, alanine, pyruvate, valine, glucose, proline, leucine or isoleucine.

2. A method as claimed in claim 1 of determining the fertilization potential of an ooctye comprising :
(1) taking a sample of follicular fluid from an oocyte in vitro,
(2) determining the lactate and glutamine concentration of the follicular fluid,
(3) calculating the ratio of lactate concentration to glutamine concentration,
(4) comparing the ratio of lactate concentration to glutamine concentration to a standard to determine if an oocyte has an increased potential for fertilisation.

3. A method as claimed in claim 1 or 2 wherein the standard is either an average ratio based on the ratio of concentrations from a plurality of oocytes which are not capable of fertilisation, or an average ratio based on the ratio of concentrations from a plurality of oocytes which are capable of fertilisation.

4. A method as claimed in any of claims 1 to 3 wherein the ratio of lactate concentration to glutamine concentration of an oocyte which will fertilise is in the range 11.13 +/- 3.21.

5. A method as claimed in claim 1 of determining whether an embryo will develop past the one cell stage comprising:
(1) taking a sample of follicular fluid from an oocyte in vitro,
(2) determining the concentration of the follicular fluid of one or more of lactate, choline/phospho-choline, alanine, pyruvate, valine, isoleucine or glucose
(3) comparing the concentrations of lactate, choline/phospho-choline, alanine, pyruvate, valine, isoleucine or glucose to a standard, wherein a decrease in the concentration of glucose or an increase in the concentration of lactate or choline/phospho-choline or alanine or pyruvate or valine or isoleucine when compared to the standard, is indicative of an oocyte that will develop past the one cell stage.

6. A method as claimed in claim 1 or 5 wherein the standard is either an average concentration based on the concentrations from a plurality of embryos which will develop past the one cell stage, or an average concentration based on the concentrations from a plurality of embryos which will not develop past the one cell stage..

7. A method as claimed in claim 1, 5 or 6 wherein the concentration of lactate, choline/phospho-choline, alanine, pyruvate, valine, isoleucine or glucose of an embryo which will develop past the one cell stage are as shown in Table 3.

8. A method as claimed in claim 1 of determining whether an embryo will implant comprising:
(1) taking a sample of follicular fluid from an oocyte in vitro,
(2) determining the concentration of the follicular fluid of one or more of glucose, proline, lactate, leucine, isoleucine or glutamine,
(3) comparing the glucose, proline, lactate, leucine, isoleucine or glutamine concentrations to a standard,wherein a decrease in the concentration of glucose or glutamine or an increase in the concentration of proline or lactate or leucine or isoleucine when compared to the standard, is indicative of a positive pregnancy outcome.

9. A method as claimed in claim 1 or 8 wherein the standard is either an average concentration based on the concentrations from a plurality of embryos which will implant, or an average concentration based on the concentrations from a plurality of embryos which will not will implant.

10. A method as claimed in claim 1, 8 or 9 wherein the concentration of glucose, proline, lactate, leucine or isoleucine of an embryo that will implant are as shown in Table 2 and the concentration of glutamine of an embryo that will implant is as shown in Table 5.

11. A diagnostic kit for increasing the success rate of IVF comprising reagents for the determination of the lactate to glutamine concentration ratio or the concentrations of lactate, glutamine, choline/phospho-choline, alanine, pyruvate, valine, glucose, proline, leucine or isoleucine.

12. Use of the concentration of one or more of the metabolites lactate, glutamine, choline/phospho-choline, alanine, pyruvate, valine, glucose, proline, leucine or isoleucine in a method of determining fertilisation potential of an embryo and/or of the ability of the embryo to develop past one cell stage and/or of an embryo's potential to implant.

13. A method of increasing the success rate of IVF comprising selection of an oocyte for fertilisation or of an embryo for implantation substantially as described herein with reference to the Examples and/or the accompanying drawings.
